# EUROPEAN PATENT APPLICATION

(11) **EP 4 517 322 A1**
(43) Date of publication of application: **05.03.2025**
(21) Application number: 24196662.1
(22) Date of filing: 27.08.2024
(51) Int. Cl.: G01N 33/487, G01N 27/327

(54) **MEASURING DEVICE**

(30) Priority: 30.08.2023 JP 2023140576
(71) Applicant: ARKRAY, Inc., Kyoto-shi, Kyoto 601-8045 (JP)
(72) Inventor: Arima, Hiroki, Kyoto-shi, Kyoto, 602-0008 (JP); Sato, Oki, Tokyo, 107-0052 (JP); Naruse, Shun, Tokyo, 107-0052 (JP)
(74) Representative: Hoffmann Eitle

(57) **Abstract**

A measuring device (10), which measures a specimen by using a sheet-shaped sensor (50), includes: a housing (20); a guiding surface (40) which is one end surface of the housing (20) and which is surrounded by peripheral edges (41); and an insertion port (30) which is formed in the guiding surface (40) at a position that is distant from all of the peripheral edges (41), and into which the sensor (50) is inserted, wherein the guiding surface (40) is recessed continuously from the peripheral edges (41) toward the insertion port (30).

## Description

### BACKGROUND

### Technical Field

The present application relates to a measuring device having an insertion port through which a sensor for measurement is inserted into a housing.

### Related Art

Japanese National Phase Publication (JP-A) No. 2014-526705 discloses a specimen measuring device at which a sensor insertion port at a side surface is a given rectangular shape.

### SUMMARY

If a periphery of an insertion port is flat, when foreign matter sticks to edges of the insertion port and a sensor is inserted into the insertion port, there is a risk that the foreign matter will enter into an interior of the measuring device.

One aspect of the present disclosure is a measuring device that measures a specimen by using a sheet-shaped sensor, the measuring device including: a housing; a guiding surface which is one end surface of the housing and which is surrounded by peripheral edges; and an insertion port which is formed in the guiding surface at a position that is distant from all of the peripheral edges, and into which the sensor is inserted, wherein the guiding surface is recessed continuously from the peripheral edges toward the insertion port.

In accordance with an embodied aspect of the present disclosure, since the insertion port is provided deep in the recessed guiding surface, even if foreign matter sticks to peripheral edges of the guiding surface, it is difficult for the foreign matter to stick to the edges of the insertion port. Further, since the guiding surface is recessed continuously from the peripheral edges, the guiding surface serves as a guide when inserting a sensor into the insertion port.

### BRIEF DESCRIPTION OF THE DRAWINGS

Exemplary embodiments will be described in detail based on the following figures, wherein:
Fig. 1 is a perspective view of a measuring device of a first embodiment;
Fig. 2 is a front view of the measuring device of Fig. 1;
Fig. 3 illustrates a state in which the measuring device of Fig. 1 has been placed on a flat surface, in a cross-sectional view that schematically illustrates the interior;
Fig. 4 is a plan view of a sensor that is to be inserted into the measuring device of the present disclosure;
Fig. 5 is a perspective view illustrating a state in which the sensor of Fig. 4 is inserted in the measuring device of Fig. 1;
Fig. 6 is a perspective view of a measuring device of a second embodiment;
Fig. 7 is a cross-sectional view schematically illustrating the interior of the measuring device of Fig. 6;
Fig. 8 is a perspective view of a measuring device of a third embodiment;
Fig. 9 is a front view of the measuring device of Fig. 8;
Fig. 10 is a right side view of the measuring device of Fig. 8;
Fig. 11 is a rear view of the measuring device of Fig. 8;
Fig. 12 is a bottom view of the measuring device of Fig. 8;
Fig. 13 is a perspective view illustrating a state in which the sensor of Fig. 4 is inserted in the measuring device of Fig. 8;
Fig. 14 is a bottom view schematically illustrating the function of an ejector in the measuring device of Fig. 8;
Fig. 15 is a cross-sectional view schematically illustrating the function of the ejector in the measuring device of Fig. 8;
Fig. 16 is a perspective view of a measuring device of a fourth embodiment;
Fig. 17 is a plan view of the measuring device of Fig. 16;
Fig. 18 is a side view of the measuring device of Fig. 16; and
Fig. 19 is a perspective view of a measuring device of a fifth embodiment.

### DETAILED DESCRIPTION

A measuring device of the present embodiment is a measuring device that measures a specimen by using a sheet-shaped sensor, and includes a housing, and an insertion port formed in one end of the housing and having a width such that the sensor can be inserted therein. A guiding surface that is the surface in which the insertion port is formed is recessed continuously from peripheral edges toward the insertion port.

A specimen mentioned here is, for example, a bodily fluid collected from an organism, and blood and urine are specific examples thereof. Measuring is performed for, e.g., a component contained in the specimen. In a case in which the specimen is blood, the component to be measured is blood sugar, hemoglobin, hemoglobin A1c, ketone or the like. In a case in which the specimen is urine, the component to be measured is urinary sugar, bilirubin, urinary protein or the like.

An insertion port into which a sensor is inserted is formed in the housing of the measuring device. The insertion port is formed in a guiding surface that is one end surface of the housing. The guiding surface is surrounded by peripheral edges. The insertion port is formed at a position that is distant from all of the peripheral edges. Due to the insertion port being formed in this way, even if foreign matter such as refuse or the like sticks to the peripheral edges, it is difficult for the foreign matter to stick to the insertion port that is distant from the peripheral edges. Further, the guiding surface is recessed continuously from the peripheral edges toward the insertion port. Due to the guiding surface being recessed in this way, at the time when a sensor is inserted into the insertion port, even if the tip of the sensor abuts a vicinity of the insertion port, the sensor is guided to the insertion port by the guiding surface that is recessed continuously. Accordingly, it is easy to insert the sensor into the measuring device.

It is preferable that the insertion port be formed at a position including the center of the guiding surface. Due to the insertion port being formed in this way, at the time when a sensor is inserted into the insertion port, the sensor is guided to the insertion port by the guiding surface that is recessed continuously, regardless of what position of the guiding surface the tip of the sensor abuts.

Note that the sensor has a strip shape, and it is preferable that the width of the insertion port correspond to the short-side length of the sensor, and that the height length of the insertion port correspond to the thickness of the sensor. Since the sensor is formed in this way, it is easy to make the insertion direction of the sensor match the shape of the insertion port. Further, it is easy to insert one end of the sensor into the insertion port while grasping another end by the fingers.

It is preferable for the guiding surface to be a convex surface. Due to the guiding surface being formed as a convex surface, the slope becomes steep from the side edges toward the insertion port, and therefore, the sensor can be quickly inserted into the insertion port.

It is preferable that the housing is formed so as to be free-standing and that the guiding surface is the surface facing the placement surface at the time when the housing stands alone. If the housing is made to be free-standing, it can be assumed that foreign matter such as refuse or dust on the placement surface will contact the surface of the housing that contacts the placement surface, i.e., the peripheral edges. In this case, because the guiding surface, which is the surface facing the placement surface, is recessed continuously from the peripheral edges toward the insertion port, even if foreign matter contacts the peripheral edges, the foreign mater does not contact the insertion port that is at the inner side of the peripheral edges.

At the measuring device of the present embodiment, it is preferable that recesses, which are sunken inward, are formed in the housing at the both ends, which run along the width direction of the insertion port, at the side opposite the insertion port. Due to such recesses being formed, when the housing is grasped by the hand opposite to the hand that is grasping the sensor, the fingers can catch on the both recesses, and therefore, it is possible to prevent the housing from being mistakenly dropped at the time of inserting a sensor.

It is preferable that the housing is a rectangular parallelepiped shape, and that these recesses are formed in the pair of surfaces that correspond to the both ends in the width direction of the insertion port, with respect to the surface at which the insertion port is positioned. Namely, by making the direction in which the recesses are provided coincide with the width direction, the housing can grasped stably at the time of inserting a sensor.

Note that it is preferable that the entireties of the aforementioned pair of surfaces are formed as recesses. Due to these surfaces being formed in this way, the housing can be supported by the fingers stably regardless of which places of the both surfaces, which are positioned in the width direction of the sensor, are grasped.

Note that it is preferable that the side surfaces of the entire periphery of the housing are formed as recesses. Due to the entire periphery being formed in this way, it is possible to grasp not only the both surfaces in the width direction of the sensor, but also the both surfaces in the longitudinal direction of the sensor.

Here, it is preferable that the recess is a concave surface that is formed in a shape in which the center along the longitudinal direction is sunken in the shape of a valley and each concave surface gradually rises from the center toward the top surface and the bottom surface. Due to the recesses being formed in this way, it is easy to insert fingertips into the sunken valleys of the recesses. Moreover, because the recesses are concave surfaces, it is easy for fingertips to fit into the curved surfaces.

Note that the shape and the significance of the sensor are as described above.

Embodiments of the present disclosure are described hereinafter with reference to the drawings. Reference numerals used in common in the respective drawings indicate the same portions unless otherwise stated. Further, the respective members and regions depicted in the drawings are merely illustrated schematically, and the sizes and positional relationships of actual products are not necessarily accurately expressed thereby.

### (1) First Embodiment

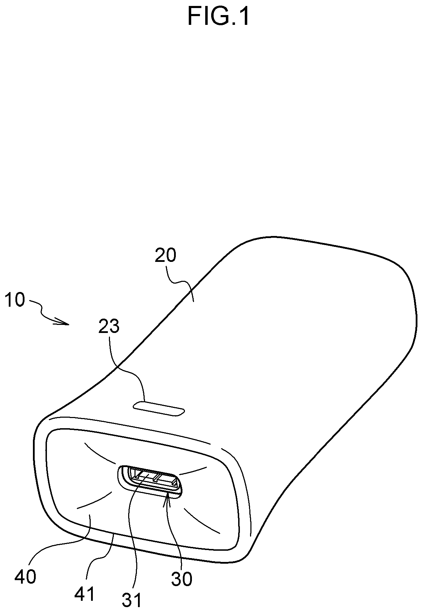

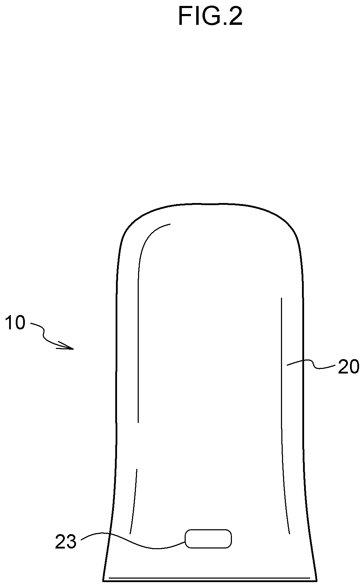

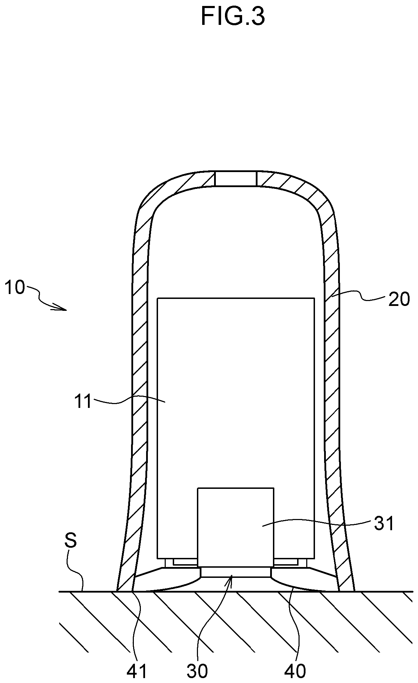

Fig. 1 and Fig. 2 illustrate a measuring device 10 of a first embodiment in a perspective view and a front view, respectively. Fig. 3 illustrates the measuring device 10 that is in a state of having been placed on a placement surface S, while illustrating the internal structure in a schematic cross-section.

### (1-1) Structure

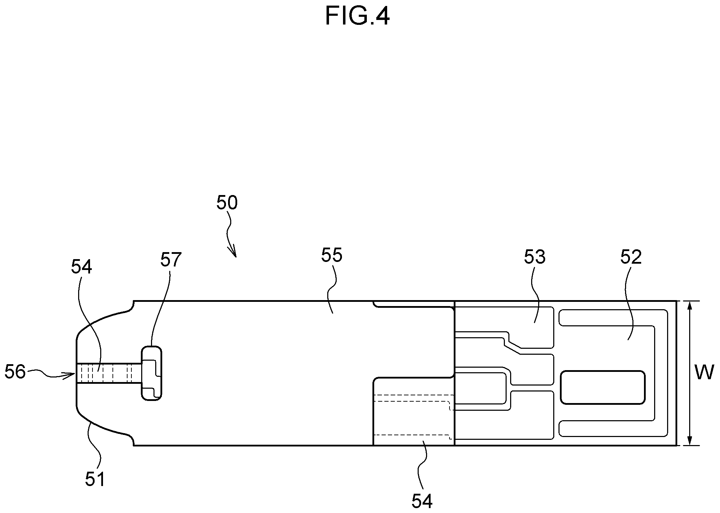

The measuring device 10 of the first embodiment is a device that measures a specimen by using a sheet-shaped sensor 50 (see Fig. 4) that is described later. The measuring device 10 of the first embodiment has a housing 20, and a guiding surface 40 that is one end surface of the housing 20 and that is surrounded by peripheral edges 41. An insertion port 30 is formed in the guiding surface 40 at a position that is distant from all of the peripheral edges 41. The sensor 50 is inserted in the insertion port 30. As illustrated in Fig. 1 and Fig. 3, the guiding surface 40, which is the surface in which the insertion port 30 is formed, is recessed continuously from the peripheral edges 41 toward the insertion port 30. In other words, the guiding surface 40 widens continuously from the insertion port 30 toward the peripheral edges 41. The insertion port 30 is formed at a position including the center of the guiding surface 40. As illustrated in Fig. 3, the guiding surface 40 is formed as a convex surface. Hereinafter, the side of the measuring device 10 at which the guiding surface 40 is formed is called the "front side", and the opposite side is called the "rear side".

Specifically, the housing 20 is formed substantially in a rectangular parallelepiped shape whose ridgelines and corners are rounded. One end surface of the rectangular parallelepiped shape that is the housing 20 is formed as the guiding surface 40. The insertion port 30 is formed at the center of the guiding surface 40. In other words, the insertion port 30 is formed at a position that is distant from all of the peripheral edges 41 of the guiding surface 40. The width direction length, which runs along the long sides of the guiding surface 40, of the insertion port 30 is formed to be larger than the height direction length that runs along the short sides. A switch 23 is provided at the top surface of the housing 20 in a vicinity of the guiding surface 40.

A portion of a sensor supporting portion 31 that is at the interior of the housing 20 is visible from the insertion port 30. As illustrated in Fig. 3, the sensor supporting portion 31 is connected to a printed circuit board (PCB) 1 1 that is incorporated into the housing 20, and is the portion contacted by the sensor 50 that has been inserted from the insertion port 30. An electrical circuit that amplifies electrical signals, an analyzing section that analyzes a concentration of the specimen, a transmitter that transmits the analyzed specimen concentration to the exterior as data, and the like, as well as a power source that drives the electrical circuit, the analyzing section, the transmitter and the like, and a power source installation portion for supplying electric power (none of these are illustrated), are provided at the PCB 11. Operation is started due to electric power being supplied to the power source installation portion from the power source such as a battery.

As illustrated in Fig. 2, the side surfaces of the housing 20 are formed so as to become slightly broader at the front side. Moreover, as illustrated in Fig. 1, the peripheral edges 41 of the guiding surface 40 are formed so as to be in the same plane. Due to the structure illustrated in Fig. 2, the measuring device 10 of the present embodiment can be made to stand alone on the placement surface S that is a flat surface such as a desk or a table, with the guiding surface 40 being made to face downward as illustrated in Fig. 3. Namely, the housing 20 of the measuring device 10 of the present embodiment is formed so as to be free standing. In other words, the guiding surface 40 is the surface that faces the placement surface S when the measuring device 10 stands alone.

Fig. 4 illustrates the sensor 50 that is used in the measuring device 10 of the present embodiment in a plan view. The sensor 50 is formed in the shape of a sheet that is substantially rectangular in plan view. At one end side of the sensor 50, the corners are rounded so as to form a shape of sloping shoulders. This portion that is formed in the shape of sloping shoulders is distal end portion 51. Hereinafter the side of the sensor 50 at which the distal end portion 51 is formed is called the "distal end side", and the side opposite thereto is called the "rear end side".

At the sensor 50, an electrode layer 53, which is formed by using a metal material such as gold (Au) or a carbon material such as carbon, is formed on a base layer 52 formed by using a synthetic resin (plastic). A portion of the electrode layer 53 is covered by a transparent spacer 54. A portion other than the spacer 54 portion is covered by a covering layer 55 that is the same material as the base layer 52. Of the electrode layer 53, the rear end side portion thereof is maintained in an exposed state in which it is not covered by the spacer 54 or the covering layer 55. A sampling port 56 is formed in the distal-most end of the distal end portion 51 as a gap formed between the electrode layer 53 and the spacer 54. An air hole 57 that passes through the covering layer 55 and the spacer 54 is provided slightly rearward of the sampling port 56. A reagent corresponding to the target substance (i.e., the substance that is intended to be measured by the measuring device 10) within the specimen is coated on the distal end side of the electrode layer 53.

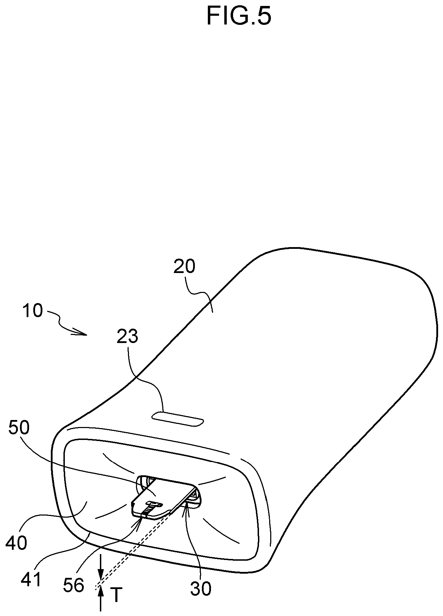

In other words, the sensor 50 has a strip shape whose length, in the horizontal direction of the drawing, is longer than the length in the vertical direction of the drawing. Further, the width direction length of the insertion port 30 illustrated in Fig. 1 corresponds to short-side direction length W (see Fig. 4) of the sensor 50. The height direction length of the insertion port 30 corresponds to thickness T of the sensor 50 illustrated in Fig. 5 that is described later.

### (1-2) Operation

As illustrated in Fig. 1 and Fig. 3, the guiding surface 40 is a convex surface and is recessed continuously from the peripheral edges 41 toward the insertion port 30. Therefore, when the measuring device 10 is stood on its own on the placement surface S, even if foreign matter on the surface of the placement surface S sticks to the peripheral edges 41, the foreign matter does not stick to the side edges of the insertion port 30.

At the time of loading the sensor 50 into the measuring device 10, first, the measuring device 10 illustrated in Fig. 1 is grasped by one hand while a region of the sensor 50 where the electrode layer 53 is not exposed is grasped by the fingers of the other hand, and the rear end side of the sensor 50 is inserted into the insertion port 30 illustrated in Fig. 1.

Further, because the guiding surface 40 is a convex surface and is recessed continuously from the peripheral edges 41 toward the insertion port 30, the electrode layer 53 side of the sensor 50 can be guided along the curved surface and inserted well into the insertion port 30 regardless of which position of the guiding surface 40 the electrode layer 53 side of the sensor 50 is made to abut.

Fig. 5 illustrates a state in which the sensor 50 is completely loaded in the measuring device 10 in a perspective view. The state illustrated in Fig. 5 is a state in which the rear end side portion of the sensor 50 is inserted within the housing 20, and the distal end side portion is exposed. In the state in which the sensor 50 is inserted in the housing 20, the electrode layer 53 at the rear end side of the sensor 50 contacts an unillustrated terminal provided at the sensor supporting portion 31 (see Fig. 3) within the housing 20, and, together with the electrical circuit formed on the PCB 11, forms a single electrical circuit.

As illustrated in Fig. 5, in the state in which the sensor 50 is inserted in the housing 20, after the switch 23 is turned on (depending on the product, there are also cases in which the power turns on by insertion of the sensor 50 is into the housing 20), when a suitable amount (e.g., one drop) of a specimen (e.g., blood) is applied to the sampling port 56, the target substance within the specimen chemically reacts with the reagent coated on the electrode at the distal end side, and the electrical signal generated by the chemical reaction is amplified through the electrical circuit. Then, the amplified electrical signal is transmitted to the aforementioned, unillustrated analyzing section. At the analyzing section, the concentration of the target substance within the specimen is analyzed on the basis of the received electrical signal(i.e., current, voltage, resistance, impedance, etc.).

### (2) Second Embodiment

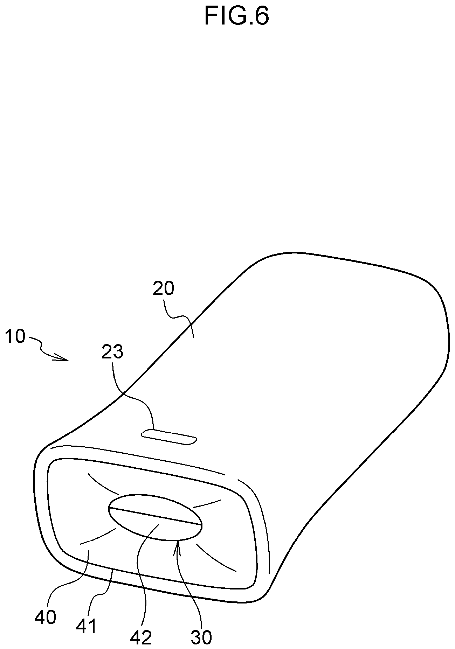

Fig. 6 illustrates the measuring device 10 of a second embodiment in a perspective view.

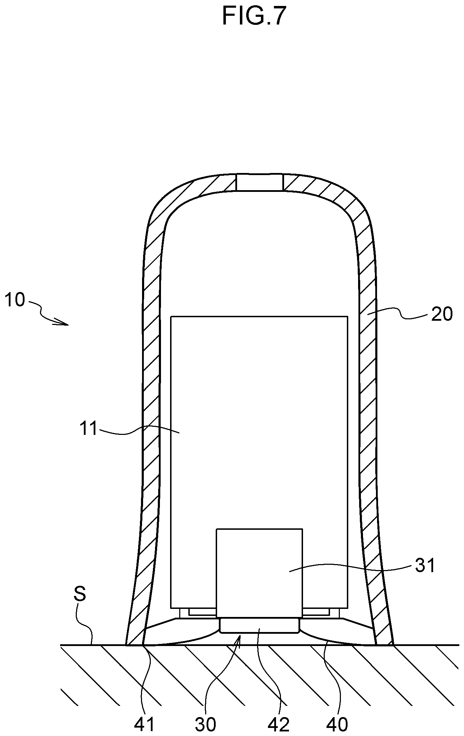

Fig. 7 illustrates a state in which the measuring device 10 of the second embodiment has been placed on the placement surface S, while illustrating the internal structure in a schematic cross-section.

### (2-1) Structure

The measuring device of the second embodiment differs from the measuring device 10 of the first embodiment with regard to the points that the guiding surface 40 is formed in the shape of a suction cup and of a flexible material such as rubber, and that the measuring device of the second embodiment has a valve body 42 at which a cut-out is formed in the center of the guiding surface 40. The external shape and internal structure other than these are similar to those of the measuring device 10 of the first embodiment.

### (2-2) Operation

As illustrated in Fig. 6 and Fig. 7, the guiding surface 40 is a convex surface and is recessed continuously from the peripheral edges 41 toward the insertion port 30. Therefore, when the measuring device 10 is stood on its own on the placement surface S as in Fig. 3, even if foreign matter on the surface of the placement surface S sticks to the peripheral edges 41, the foreign matter does not stick to the side edges of the insertion port 30.

In addition, when the housing is pushed from the free-standing state illustrated in Fig. 7 toward the placement surface S, the guiding surface 40 that serves as a suction cup sticks fast to the placement surface S due to the front side of the housing 20 flexing slightly outward due to elastic deformation. At the time when the guiding surface 40 sticks fast to the placement surface S, the air that was between the guiding surface 40 and the placement surface S is discharged into the housing through the valve body 42 that opens at the instant of the suction.

The loading of the sensor 50 into the measuring device 10, and the measuring of the specimen thereafter, are similar to those of the first embodiment.

### (3) Third Embodiment

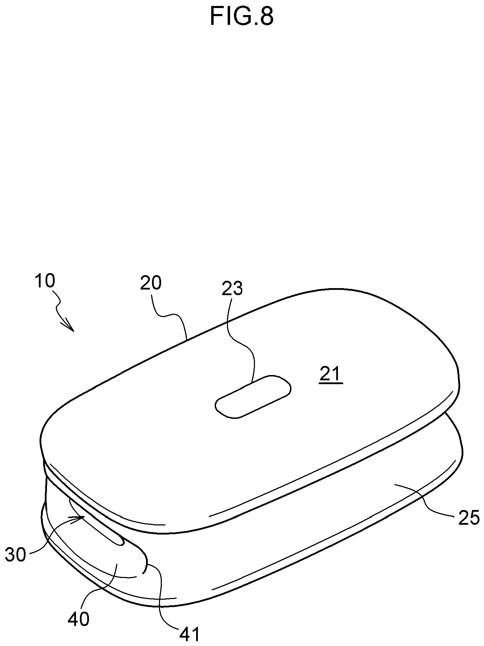

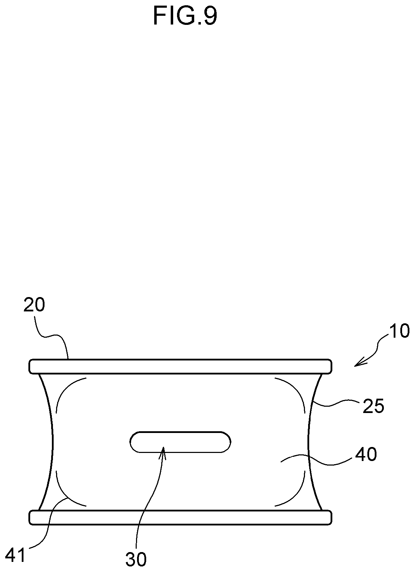

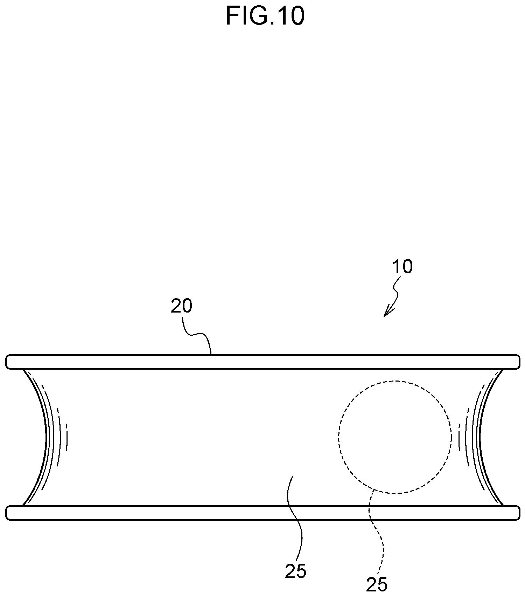

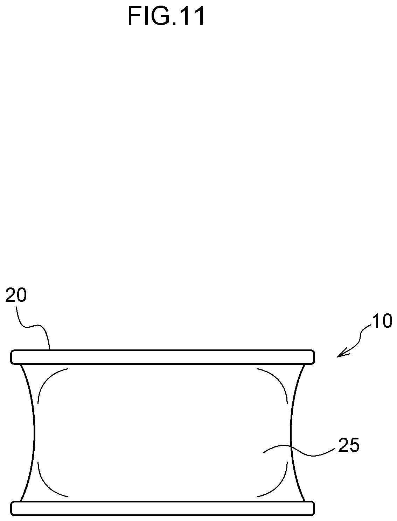

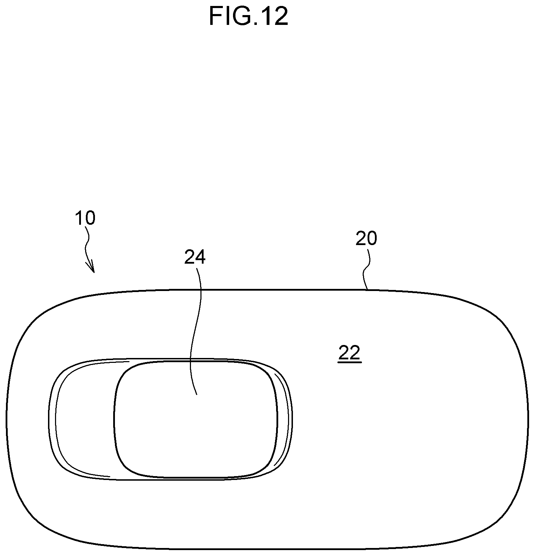

Figs. 8 to 12 illustrate the measuring device 10 of a third embodiment in a perspective view (Fig. 8), a front view (Fig. 9), a right side view (Fig. 10), a rear view (Fig. 11) and a bottom view (Fig. 12).

### (3-1) Structure

The measuring device 10 of the third embodiment measures a specimen by using the above-described, sheet-shaped sensor 50 (see Fig. 4). The measuring device 10 of the third embodiment has the housing 20, and the insertion port 30 that is formed in one end of the housing 20 and that is of a width such that the sensor 50 can be inserted therein. The side surfaces of the entire periphery of the housing 20 are formed as recesses 25. Hereinafter, the side of the measuring device 10 at which the insertion port 30 is formed is called the "front side", and the opposite side is called the "rear side".

The recesses 25 do not have to be provided over the entire periphery of the housing 20 as in the present embodiment, and may be provided only at the right side surface shown in Fig. 10 and the left side surface that has left-right symmetry thereto, and the front surface and the rear surface may be formed in planar shapes. Namely, the entireties of the pair of surfaces that correspond to the both ends in the width direction of the insertion port (i.e., the right side surface and the left side surface), with respect to the surface where the insertion port 30 is positioned (i.e., the front surface), are formed as the recesses 25. As illustrated in Fig. 9 to Fig. 11, the recess 25 is a concave surface that is formed in a shape in which the center along the longitudinal direction is sunken in the shape of a valley and each concave surface gradually rises from the center toward the top surface and the bottom surface.

Further, the recesses 25 may be formed as a pair of concave portions that are provided at the rear sides, which are shown by the dashed line in Fig. 10, of the right side surface and the left side surface that are formed in planar shapes. Namely, the recesses 25 are formed in the pair of surfaces that correspond to the both ends in the width direction of the insertion port (i.e., the right side surface and the left side surface), with respect to the surface where the insertion port 30 is positioned (i.e., the front surface).

Moreover, even if the housing 20 is not a rectangular parallelepiped shape (e.g., is a substantially cylindrical shape whose one side is trimmed to a planar shape in order to prevent rolling), the recesses 25 that are sunken inward may be formed in the housing 20 in the both ends that run along the width direction of the insertion port 30 at the side opposite the insertion port 30 (i.e., at the rear side).

Specifically, the housing 20 is a substantial rectangular parallelepiped shape that is structured from six surfaces that are a top surface (Fig. 8) and a bottom surface (Fig. 12) that are substantially rectangular and whose four corners are rounded, and a front surface (Fig. 8 and Fig. 9), a rear surface (Fig. 11) and both side surfaces (Fig. 8 and Fig. 10, the left side surface being omitted) in which the recesses 25 are formed.

In the third embodiment as well, in the same way as in the first embodiment, the guiding surface 40, which is one end surface (i.e., the front surface) of the housing 20 and is surrounded by the peripheral edges 41, is provided. The insertion port 30 is formed in the guiding surface 40 at a position that is distant from all of the peripheral edges 41. The sensor 50 is inserted into the insertion port 30. As illustrated in Fig. 8 and Fig. 9, the guiding surface 40, which is the surface in which the insertion port 30 is formed, is recessed continuously from the peripheral edges 41 toward the insertion port 30. The insertion port 30 is formed at a position including the center of the guiding surface 40. As illustrated in Fig. 8 and Fig. 9, the guiding surface 40 is formed as a convex surface.

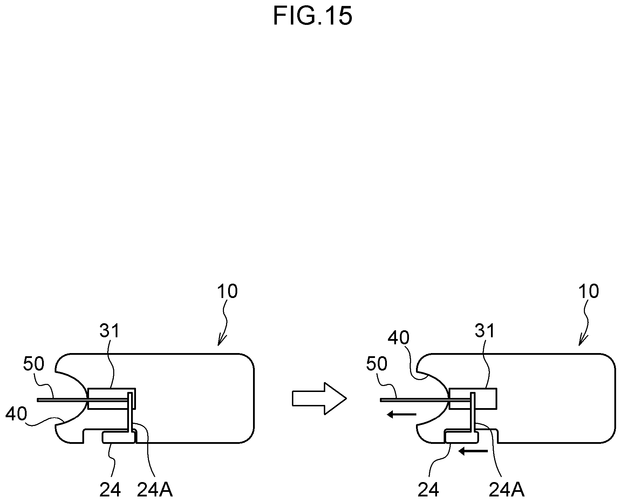

The insertion port 30 is formed such that the width direction length thereof, which runs along the longitudinal direction of the guiding surface 40, is larger than the height direction length thereof that runs along the short-side direction. The switch 23 (Fig. 8) is provided at the center of a top surface 21 of the housing 20. On the other hand, a substantially rectangular ejector 24 is provided at a position of the bottom surface of the housing 20 that is slightly forward of the center. A pushing portion 24A (see Fig. 15), which is abutted by the rear end of the inserted sensor 50, projects out into the interior of the housing 20 from the inner surface in a vicinity of the rear end of the ejector 24.

In the same way as in the first embodiment, a portion of the unillustrated sensor supporting portion 31 (see Fig. 3) that is incorporated into the housing 20 is visible at the interior of the insertion port 30. As illustrated in Fig. 3, the sensor supporting portion 31 is connected to the unillustrated PCB 11 (see Fig. 3) that is incorporated into the housing 20, and is the portion contacted by the sensor 50 that has been inserted from the insertion port 30. An electrical circuit that amplifies electrical signals, an analyzing section that analyzes a concentration of the specimen, a transmitter that transmits the analyzed specimen concentration to the exterior as data, and the like, as well as a power source that drives the electrical circuit, the analyzing section, the transmitter and the like, and a power source installation portion for supplying electric power (none of these are illustrated), are provided at the PCB 11. Operation is started due to electric power being supplied to the power source installation portion from the power source such as a battery.

The sensor 50 illustrated in Fig. 4 is used in the measuring device 10 of the third embodiment as well. The structure of and the method of using the sensor 50 are similar to those of first embodiment.

### (3-2) Operation

As illustrated in Fig. 8 and Fig. 9, the guiding surface 40 is recessed continuously from the peripheral edges 41 toward the insertion port 30. Therefore, even if foreign matter sticks to the peripheral edges 41, it is difficult for the foreign matter to stick to the side edges of the insertion port 30.

At the time of loading the sensor 50 into the measuring device 10, first, the recesses 25 of the measuring device 10 illustrated in Fig. 1 are grasped by one hand while a region of the sensor 50 where the electrode layer 53 is not exposed is grasped by the fingers of the other hand, and the rear end side of the sensor 50 is inserted into the insertion port 30 illustrated in Fig. 8 and Fig. 9. Due to fingers grasping the recesses 25, it is possible to prevent the measuring device 10 from being mistakenly dropped at the time of insertion. Further, because the width direction is clearly larger than the height direction (or the thickness direction) at both the insertion port 30 and the sensor 50, the sensor 50 can be inserted in the correct direction.

In the third embodiment, because the guiding surface 40 is recessed continuously from the peripheral edges 41 toward the insertion port 30, the electrode layer 53 side of the sensor 50 can be guided along the curved surface and inserted well into the insertion port 30 regardless of which position of the guiding surface 40 the electrode layer 53 side of the sensor 50 is made to abut.

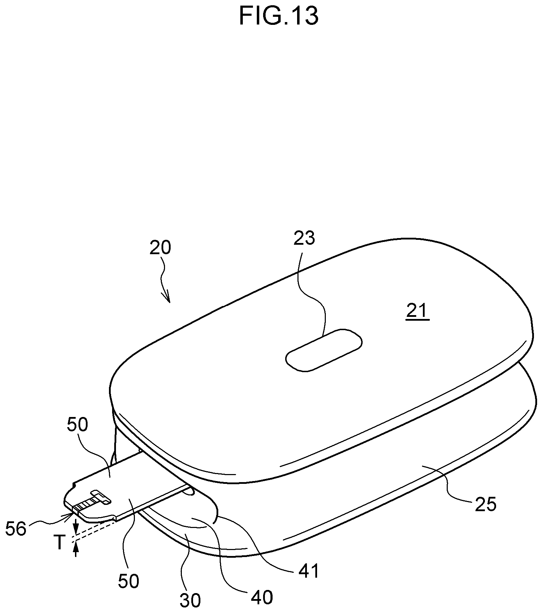

Fig. 13 illustrates a state in which the sensor 50 is completely loaded in the measuring device 10 in a perspective view. The state illustrated in Fig. 13 is a state in which the rear end side portion of the sensor 50 is inserted within the housing 20, and the distal end side portion is exposed. In the state in which the sensor 50 is inserted in the housing 20, the electrode layer 53 at the rear end side of the sensor 50 contacts an unillustrated terminal provided at the sensor supporting portion 31 (see Fig. 3) within the housing 20, and, together with the electrical circuit formed on the PCB 11, forms a single electrical circuit.

As illustrated in Fig. 13, in the state in which the sensor 50 is inserted in the housing 20, after the switch 23 is turned on (depending on the product, there are also cases in which the power turns on by insertion of the sensor 50 into the housing 20), when a suitable amount (e.g., one drop) of the specimen (e.g., blood) is applied to the sampling port 56, the target substance within the specimen chemically reacts with the reagent coated on the electrode at the distal end side, and the electrical signal generated by the chemical reaction is amplified through the electrical circuit. Then, the amplified electrical signal is transmitted to the unillustrated analyzing section at the interior of the housing 20. At the analyzing section, the concentration of the target substance within the specimen is analyzed on the basis of the received electrical signal.

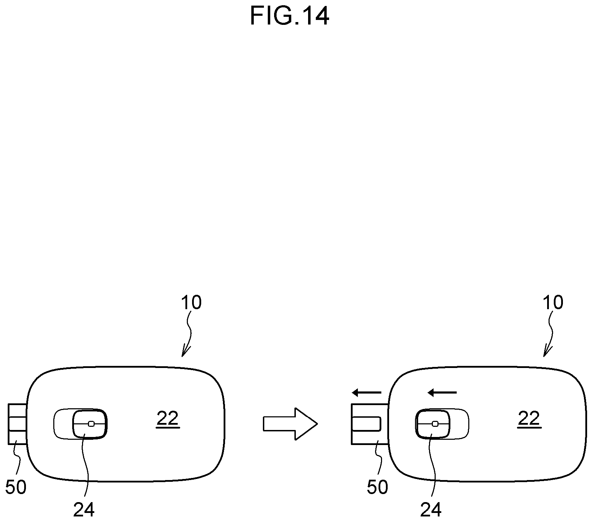

After measurement of the specimen is completed, when the ejector 24 is moved forward from its initial position illustrated at the left sides of Fig. 14 and Fig. 15, the pushing portion 24A pushes the rear end of the sensor 50 forward, and the sensor 50 can be removed.

### (4) Fourth Embodiment

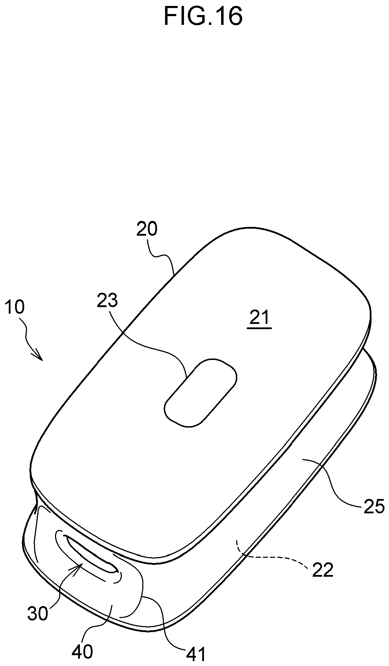

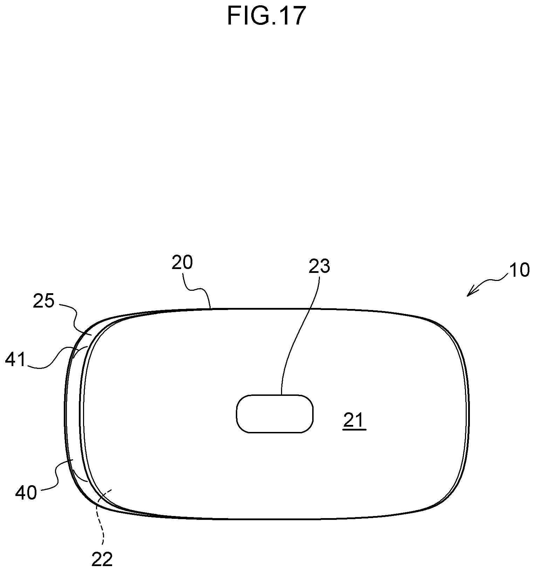

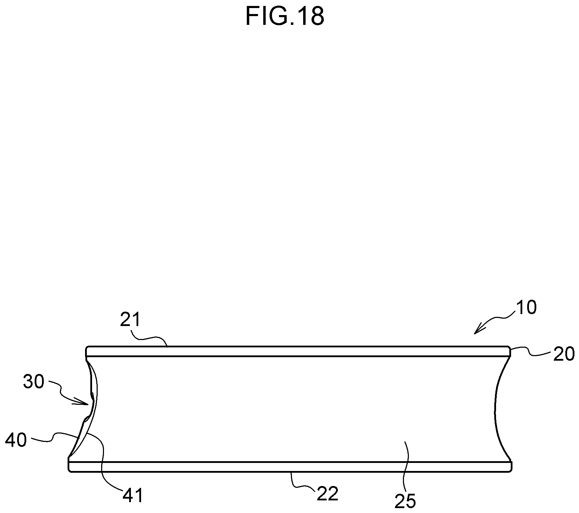

Fig. 16 to Fig. 18 illustrate the measuring device 10 of a fourth embodiment in a perspective view (Fig. 16), a plan view (Fig. 17) and a side view (Fig. 18).

In the present embodiment, because bottom surface 22 protrudes out further forward than the top surface 21, when the measuring device 10 is viewed from slightly above which is the usual viewpoint, it is easy to see the insertion port 30, and accordingly, it is easy to insert the sensor 50.

The external shape, internal structure, and operation of the present embodiment other than these are similar to those of the measuring device 10 of the third embodiment.

### (5) Fifth Embodiment

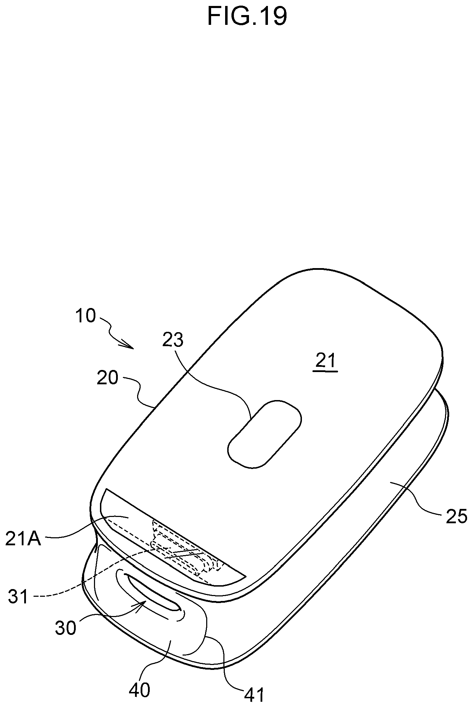

Fig. 19 illustrates the measuring device 10 of a fifth embodiment in a perspective view. A transparent window 21A, which is formed of a transparent or semitransparent material, is formed at a region in the vicinity of the front side of the top surface 21. The sensor supporting portion 31 at the interior of the housing 20 can be seen through the transparent window 21A. Due to the formation of the transparent window 21A, at the time of viewing the measuring device 10 from slightly above which is the usual viewpoint, it is easy to see the sensor supporting portion 31 that is at the deep rear of the insertion port 30, and accordingly, it is easy to insert the sensor 50.

The external shape, internal structure and operation of the present embodiment other than these are similar to those of the measuring device 10 of the third embodiment.

Note that preferable forms of the present disclosure are additionally presented hereinafter.

### (Note 1)

A measuring device that measures a specimen by using a sheet-shaped sensor, the measuring device including:
a housing;
a guiding surface which is one end surface of the housing and which is surrounded by peripheral edges; and
an insertion port which is formed in the guiding surface at a position that is distant from all of the peripheral edges, and into which the sensor is inserted,
wherein the guiding surface is recessed continuously from the peripheral edges toward the insertion port.

### (Note 2)

The measuring device of Note 1, wherein the insertion port is formed at a position including a center of the guiding surface.

### (Note 3)

The measuring device of Note 1 or Note 2, wherein
the sensor has a strip shape,
a width direction length of the insertion port corresponds to a short-side direction length of the sensor, and
a height direction length of the insertion port corresponds to a thickness of the sensor.

### (Note 4)

The measuring device of any one of Note 1 to Note 3, wherein the guiding surface is a convex surface.

### (Note 5)

The measuring device of any one of Note 1 to Note 4, wherein the housing is formed to be free-standing, and the guiding surface faces a placement surface when the housing stands alone.

### (Note 6)

The measuring device of Note 1, wherein recesses, which are sunken inward, are formed in the housing at both ends, which run along a width direction of the insertion port, at a side opposite the insertion port.

### (Note 7)

The measuring device of Note 6, wherein the housing is a rectangular parallelepiped shape, and the recesses are formed in a pair of surfaces, which correspond to both ends in the width direction of the insertion port, with respect to a surface at which the insertion port is positioned.

### (Note 8)

The measuring device of Note 6 or Note 7, wherein entireties of the pair of surfaces are formed as the recesses.

### (Note 9)

The measuring device of any one of Note 6 through Note 8, wherein side surfaces of an entire periphery are formed as the recesses.

### (Note 10)

The measuring device of any one of Note 6 through Note 9, wherein the recesses are concave surfaces that are formed in shapes in which a center along a longitudinal direction is sunken in a shape of a valley and each concave surface gradually rises from the center toward a top surface and a bottom surface.

### (Note 11)

A measuring device that measures a specimen by using a sheet-shaped sensor, the measuring device including:
a housing; and
an insertion port formed in one end of the housing, and having a width such that the sensor can be inserted therein,
wherein recesses, which are sunken inward, are formed in the housing at both ends, which run along a width direction of the insertion port, at a side opposite the insertion port.

### (Note 12)

The measuring device of Note 11, wherein the housing is a rectangular parallelepiped shape, and the recesses are formed in a pair of surfaces, which correspond to both ends in the width direction of the insertion port, with respect to a surface at which the insertion port is positioned.

### (Note 13)

The measuring device of Note 12, wherein entireties of the pair of surfaces are formed as the recesses.

### (Note 14)

The measuring device of Note 13, wherein side surfaces of an entire periphery of the housing are formed as the recesses.

### (Note 15)

The measuring device of any one of Note 11 to Note 14, wherein the recesses are concave surfaces that are formed in shapes in which a center along a longitudinal direction is sunken in a shape of a valley and each concave surface gradually rises from the center toward a top surface and a bottom surface.

### (Note 16)

The measuring device of any one of Note 11 to Note 15, wherein
the sensor has a strip shape,
a width direction length of the insertion port corresponds to a short-side direction length of the sensor, and
a height direction length of the insertion port corresponds to a thickness of the sensor.

### Industrial Applicability

The present disclosure can be applied to measuring devices having, at a housing, an insertion port into which a sensor for measurement is inserted, such as a blood sugar measuring device.

## Claims

1. A measuring device (10) that measures a specimen by using a sheet-shaped sensor (50), the measuring device (10) comprising:
a housing (20);
a guiding surface (40) which is one end surface of the housing (20) and which is surrounded by peripheral edges (41); and
an insertion port (30) which is formed in the guiding surface (40) at a position that is distant from all of the peripheral edges (41), and into which the sensor (50) is inserted,
wherein the guiding surface (40) is recessed continuously from the peripheral edges (41) toward the insertion port (30).

2. The measuring device (10) of claim 1, wherein the insertion port (30) is formed at a position including a center of the guiding surface (40).

3. The measuring device (10) of claim 1 or claim 2, wherein:
the sensor (50) has a strip shape,
a width direction length of the insertion port (30) corresponds to a short-side direction length (W) of the sensor (50), and
a height direction length of the insertion port (30) corresponds to a thickness (T) of the sensor (50).

4. The measuring device (10) of any one of claims 1 to 3, wherein the guiding surface (40) is a convex surface.

5. The measuring device (10) of any one of claims 1 to 4, wherein the housing (20) is formed to be free-standing, and the guiding surface (40) faces a placement surface (S) when the housing (20) stands alone.

6. The measuring device (10) of any one of claims 1 to 5, wherein recesses (25), which are sunken inward, are formed in the housing (20) at both ends, which run along a width direction of the insertion port (30), at a side opposite the insertion port (30).

7. The measuring device (10) of claim 6, wherein the housing (20) is a rectangular parallelepiped shape, and the recesses (25) are formed in a pair of surfaces, which correspond to both ends in the width direction of the insertion port (30), with respect to a surface at which the insertion port (30) is positioned.

8. The measuring device (10) of claim 7, wherein entireties of the pair of surfaces are formed as the recesses (25).

9. The measuring device (10) of claim 8, wherein side surfaces of an entire periphery of the housing (20) are formed as the recesses (25).

10. The measuring device (10) of any one of claims 6 to 9, wherein the recesses (25) are concave surfaces that are formed in shapes in which a center along a longitudinal direction is sunken in a shape of a valley and each concave surface gradually rises from the center toward a top surface (21) and a bottom surface (22).
